# EUROPEAN PATENT APPLICATION

(11) **EP 4 015 484 A1**
(43) Date of publication of application: **22.06.2022**
(21) Application number: 21155454.8
(22) Date of filing: 05.02.2021
(51) Int. Cl.: C04B 35/486, A61C 13/00, A61K 6/818, A61K 6/822, C04B 35/488

(54) **PINK COLORED PRE-SINTERED OR FULLY-SINTERED BLANK**

(30) Priority: 15.12.2020 US 202063125445 P
(71) Applicant: Dentsply Sirona Inc., York, PA 17401 (US); DeguDent GmbH, 63457 Hanau (DE)
(72) Inventor: FECHER, Stefan, 63867 Johannesberg (DE); VÖLKL, Lothar, 63773 Goldbach (DE); AMMON, Daniel, Webster, NY 14580 (US)
(74) Representative: Singh, Tajeshwar

(57) **Abstract**

The present invention is related to a pink colored pre-sintered or fully-sintered blank for use for the production of a dental restoration, such as a denture base of a full or a partial denture, a partial denture, or an implant supported denture , consisting of a ceramic material which comprises zirconium dioxide doped with yttrium oxide (Y₂O₃), calcium oxide (CaO), magnesium oxide (MgO) and/or cerium oxide (CeO₂), wherein the pink colored blank comprises 2 to 25 wt%, preferably 4 to 17 wt%, and more preferably 5 to 12 wt%, erbium oxide.

## Description

### Field of the Invention

The present invention relates to a pink colored pre-sintered or fully-sintered blank for use for the production of a dental restoration; such as a denture base of a full or a partial denture, a partial denture, or an implant supported denture; consisting of a ceramic material which comprises zirconium dioxide doped with yttrium oxide (Y₂O₃), calcium oxide (CaO), magnesium oxide (MgO) and/or cerium oxide (CeO₂).

### Background of the Invention

US 8 936 848 B2 discloses a blank of zirconium dioxide that is used for the preparation of a tooth replacement and comprises a number of layers of different chemical compositions. The individual layers thereby have different percentages of yttrium oxide.

A body of zirconium dioxide exhibits a decrease or increase in chromaticity along a straight line in the L*a*b* color space.

A blank of zirconium dioxide for the preparation of dental objects in accordance with WO 2014/062375 A1 has at least two material regions which have different proportions of tetragonal and cubic crystal phases, wherein in one of the regions the ratio is greater than 1 and in the other region the ratio is lower than 1.

EP 2 371 344 A1 relates to a ceramic body which is enriched with a stabilizing agent from the surface to a desired depth.

Zirconium dioxide is used as a ceramic material to produce dental restorations. A framework can be milled, for example, from a blank of zirconium dioxide and can then be sintered. In the following processing stages, a veneer is applied manually to the framework, wherein at least one incisor material is applied manually and fused. All of these process steps are time-consuming.

Even when many ceramic materials for dental restorations based on a zirconium dioxide system have already been investigated, it has been found very challenging up to the present to produce a specific ceramic base material, which is able to provide a pink color having a high similarity to human gingiva.

The provision of such a pink colored ceramic material and methods for producing them are more and more required by the dental industry for dental restorations, such as for the production of a denture base of a full or a partial denture, a partial denture, or an implant supported denture.

### Objective of the present Invention

In view of the prior art, it was thus an object of the present invention to provide a specific pink colored ceramic material for dental restoration applications wherein said pink colored ceramic material shall be as similar as possible to human gingiva.

### Summary of the Invention

These objects and further objects which are not stated explicitly but are immediately derivable or discernible from the connections discussed herein by way of introduction are achieved by a pink colored pre-sintered or fully-sintered blank having all features of claim 1. Appropriate modifications to the inventive pink colored pre-sintered or fully-sintered blank are protected in dependent claims 2 to 11. Further, claim 12 comprises the use of such a pink colored pre-sintered or fully-sintered blank for the production of a denture base of a full or a partial denture, a partial denture, or an implant supported denture.

The present invention accordingly provides a pink colored pre-sintered or fully-sintered blank for use for the production of a dental restoration, such as a denture base of a full or a partial denture, a partial denture, or an implant supported denture, consisting of a ceramic material which comprises zirconium dioxide doped with yttrium oxide (Y₂O₃), calcium oxide (CaO), magnesium oxide (MgO) and/or cerium oxide (CeO₂),
characterized in that
the pink colored blank comprises 2 to 25 wt%, preferably 4 to 17 wt%, and more preferably 5 to 12 wt%, erbium oxide.

It is thus possible in an unforeseeable manner to provide a specific pink colored ceramic material for dental restoration applications wherein said pink colored ceramic material is similar to human gingiva.

### Detailed Description of the Invention

The expression "substantially free" means in the context of the present invention a concentration of less than 0.0005 weight percent, preferably less than 0.0003 weight percent, and more preferably less than 0.0001 weight percent.

In one preferred embodiment, the pink colored blank is substantially, preferably completely, free of any oxides of the elements Pr, Al, Fe, Co, Ni, Ti, V, Cr, Cu, Mn, and Tb.

In one alternative embodiment thereto, the pink colored blank further comprises at least one oxide of the elements Mn, Al, Fe, Co, Tb and Pr.

In a first preferred embodiment thereof, the pink colored blank further comprises between 0.0005 and 0.02 wt%, preferably between 0.0005 and 0.01 wt%, and more preferably between 0.0005 and 0.005 wt% of an oxide of the element Mn; and/or the pink colored blank further comprises between 0.0005 and 0.1 wt%, preferably between 0.0005 and 0.07 wt%, and more preferably between 0.0005 and 0.05 wt% of an oxide of the element Co.

In a second preferred embodiment thereof, the pink colored blank further comprises between 0.0005 and 0.3 wt%, preferably between 0.0005 and 0.2 wt%, and more preferably between 0.0005 and 0.1 wt% of an oxide of the element Al; and/or the pink colored blank further comprises between 0.0005 and 0.1 wt%, preferably between 0.0005 and 0.06 wt%, and more preferably between 0.0005 and 0.03 wt% of an oxide of the element Fe.

In a third preferred embodiment thereof, the pink colored blank further comprises between 0.0005 and 0.5 wt%, preferably between 0.0005 and 0.3 wt%, and more preferably between 0.0005 and 0.2 wt% of an oxide of the element Tb; and/or the pink colored blank further comprises between 0.0005 and 0.5 wt%, preferably between 0.0005 and 0.3 wt%, and more preferably between 0.0005 and 0.2 wt% of an oxide of the element Pr.

In a fourth preferred embodiment thereof, the pink colored blank comprises 2 to 25 wt%, preferably 4 to 17 wt%, and more preferably 5 to 12 wt%, erbium oxide; wherein the pink colored blank further comprises between 0.0005 and 0.1 wt%, preferably between 0.0005 and 0.06 wt%, and more preferably between 0.0005 and 0.03 wt% of an oxide of the element Fe; and wherein the pink colored blank is substantially, preferably completely, free of any oxides of the elements Pr, Al, Co, Ni, Ti, V, Cr, Cu, Mn, and Tb.

In a fifth preferred embodiment thereof, the pink colored blank comprises 2 to 25 wt%, preferably 4 to 17 wt%, and more preferably 5 to 12 wt%, erbium oxide; wherein the pink colored blank further comprises between 0.0005 and 0.1 wt%, preferably between 0.0005 and 0.06 wt%, and more preferably between 0.0005 and 0.03 wt% of an oxide of the element Fe; wherein the pink colored blank further comprises between 0.0005 and 0.1 wt%, preferably between 0.0005 and 0.07 wt%, and more preferably between 0.0005 and 0.05 wt% of an oxide of the element Co; and wherein the pink colored blank is substantially, preferably completely, free of any oxides of the elements Pr, Al, Ni, Ti, V, Cr, Cu, Mn, and Tb.

In one embodiment, the zirconium dioxide is doped with yttrium oxide, wherein the percentage of yttrium oxide in the pink colored blank is between 1 wt% and 15 wt%, preferably between 2 wt% and 11 wt%, and more preferably between 2.5 wt% and 10 wt%.

In one embodiment, the pink colored blank possesses an optical appearance, which is characterized by L, a, and b values, which have been measured by a Vita Easyshade digital spectrophotometer.

In a preferred embodiment thereof, the pink colored blank possesses an L value ranging from 55 to 70, an a value ranging from 16 to 25, and a b value ranging from 14 to 35.

The above pink colored pre-sintered or fully-sintered blank of the present invention has been found suitable for the production of a denture base of a full or a partial denture, a partial denture, or an implant supported denture.

The present invention thus addresses the problem of providing a specific pink colored ceramic material for dental restoration applications wherein said pink color ceramic material shall be as similar as possible to human gingiva.

While the principles of the invention have been explained in relation to certain particular embodiments, and are provided for purposes of illustration, it is to be understood that various modifications thereof will become apparent to those skilled in the art upon reading the specification. Therefore, it is to be understood that the invention disclosed herein is intended to cover such modifications as fall within the scope of the appended claims. The scope of the invention is limited only by the scope of the appended claims.

## Claims

1. A pink colored pre-sintered or fully-sintered blank for use for the production of a dental restoration; such as a denture base of a full or a partial denture, a partial denture, or an implant supported denture ; consisting of a ceramic material which comprises zirconium dioxide doped with yttrium oxide (Y₂O₃), calcium oxide (CaO), magnesium oxide (MgO) and/or cerium oxide (CeO₂),
**characterized in that**
the pink colored blank comprises 2 to 25 wt%, preferably 4 to 17 wt%, and more preferably 5 to 12 wt%, erbium oxide.

2. The blank according to claim 1,
**characterized in that**
the pink colored blank is substantially, preferably completely, free of any oxides of the elements Pr, Al, Fe, Co, Ni, Ti, V, Cr, Cu, Mn, and Tb.

3. The blank according to claim 1,
**characterized in that**
the pink colored blank further comprises at least one oxide of the elements Mn, Al, Fe, Co, Tb and Pr.

4. The blank according to claim 3,
**characterized in that**
the pink colored blank further comprises between 0.0005 and 0.02 wt%, preferably between 0.0005 and 0.01 wt%, and more preferably between 0.0005 and 0.05 wt% of an oxide of the element Mn; and/or the pink colored blank further comprises between 0.0005 and 0.1 wt%, preferably between 0.0005 and 0.07 wt%, and more preferably between 0.0005 and 0.05 wt% of an oxide of the element Co.

5. The blank according to claim 3 or 4,
**characterized in that**
the pink colored blank further comprises between 0.0005 and 0.3 wt%, preferably between 0.0005 and 0.2 wt%, and more preferably between 0.0005 and 0.1 wt% of an oxide of the element Al; and/or the pink colored blank further comprises between 0.0005 and 0.1 wt%, preferably between 0.0005 and 0.06 wt%, and more preferably between 0.0005 and 0.03 wt% of an oxide of the element Fe.

6. The blank according to one of claims 3 to 5,
**characterized in that**
the pink colored blank further comprises between 0.0005 and 0.5 wt%, preferably between 0.0005 and 0.3 wt%, and more preferably between 0.0005 and 0.2 wt% of an oxide of the element Tb; and/or the pink colored blank further comprises between 0.0005 and 0.5 wt%, preferably between 0.0005 and 0.3 wt%, and more preferably between 0.0005 and 0.2 wt% of an oxide of the element Pr.

7. The blank according to claim 1,
**characterized in that**
the pink colored blank comprises 2 to 25 wt%, preferably 4 to 17 wt%, and more preferably 5 to 12 wt%, erbium oxide; wherein the pink colored blank further comprises between 0.0005 and 0.1 wt%, preferably between 0.0005 and 0.06 wt%, and more preferably between 0.0005 and 0.03 wt% of an oxide of the element Fe; and wherein the pink colored blank is substantially, preferably completely, free of any oxides of the elements Pr, Al, Co, Ni, Ti, V, Cr, Cu, Mn, and Tb.

8. The blank according to claim 1,
**characterized in that**
the pink colored blank comprises 2 to 25 wt%, preferably 4 to 17 wt%, and more preferably 5 to 12 wt%, erbium oxide; wherein the pink colored blank further comprises between 0.0005 and 0.1 wt%, preferably between 0.0005 and 0.06 wt%, and more preferably between 0.0005 and 0.03 wt% of an oxide of the element Fe; wherein the pink colored blank further comprises between 0.0005 and 0.1 wt%, preferably between 0.0005 and 0.07 wt%, and more preferably between 0.0005 and 0.05 wt% of an oxide of the element Co; and wherein the pink colored blank is substantially, preferably completely, free of any oxides of the elements Pr, Al, Ni, Ti, V, Cr, Cu, Mn, and Tb.

9. The blank according to one of the preceding claims,
**characterized in that**
the zirconium dioxide is doped with yttrium oxide, wherein the percentage of yttrium oxide in the pink colored blank is between 1 wt% and 15 wt%, preferably between 2 wt% and 11 wt%, and more preferably between 2.5 wt% and 10 wt%.

10. The blank according to one of the preceding claims,
**characterized in that**
the pink colored blank possesses an optical appearance, which is **characterized by** L, a, and b values, which have been measured by a Vita Easyshade digital spectrophotometer.

11. The blank according to claim 10,
**characterized in that**
the pink colored blank possesses an L value ranging from 55 to 70, an a value ranging from 16 to 25, and a b value ranging from 14 to 35.

12. Use of a pink colored pre-sintered or fully-sintered blank according to claims 1 to 11 for the production of a denture base of a full or a partial denture, a partial denture, or an implant supported denture.
